# EUROPEAN PATENT APPLICATION

(11) **EP 0 657 538 A2**
(43) Date of publication of application: **14.06.1995**
(21) Application number: 94830567.7
(22) Date of filing: 07.12.1994
(51) Int. Cl.: C12N 15/82, C12N 15/13, C12P 21/08, G01N 33/53

(54) **Process to produce engineered antibodies in plants, engineered antibodies and use thereof in diagnostics and therapeutics**

(30) Priority: 10.12.1993 IT RM930818
(71) Applicant: ENEA ENTE PER LE NUOVE TECNOLOGIE, L'ENERGIA E L'AMBIENTE, I-00198 Roma (IT)
(72) Inventor: Galeffi, Patrizia, ENEA - INNO BIO AG, I-00060 S. Maria di Galeria (IT); Natali, Piergiorgio, Istituti Fisioterapici, I-00162 Roma (IT)
(74) Representative: de Simone, Domenico

(57) **Abstract**

A process to produce antibodies to be used for diagnosis and therapy purpose is described. Preferably the antibodies according to the invention recognize the HER-2 oncogene product that is present in mammary and ovary tumors.

## Description

The invention relates to a process to produce engineered antibodies in plants, antibodies and the use thereof for diagnostics and therapeutics.

More specifically, the invention relates to a process to produce single chain antibodies in transgenic plants to be used for diagnostics and therapeutics.

The production of antibodies to be used in diagnostics and therapeutics is of great importance for either basic or applied research in molecular pathology, clinical immunology, etc.

A significant requirement of the field consists of finding systems of antibody production that are able to maintain proper specificity, effectiveness and pharmakinetics characteristics, being at the same time alternative processes to classic monoclonal antibody production, because of the very restricted use of said antibodies on large scale.

Recently, biotechnology research discovered that different biologic systems (such as bacteria, fungi, cultured animal cells and transgenic animals) could be used to optimize the production and/or the purification of organic molecules and/or proteins. By means of either recombinant DNA or genetic and protein engineering techniques, as well as the more advanced (recombinant and/or engineered) antibody technique, DNA sequences could be expressed in a biological context other than the natural one.

Ward and al. (1) found that an antibody single domain, named dAbs (Variable Single Heavy Region), obtained in E. coli, maintained the ability to recognize the antigen.

Hiatt and al. (2, 3) demonstrated that murine entire antibodies could be produced in plants.

Even if a number of specific literature was published, it is universally reported that both monoclonal and engineered (4) and dispecific antibodies (5) cannot be used effectively in an in vivo therapy. Specifically, typical drawbacks are as follows: a) local and/or general adverse reactions, due to the parenteral administration of murine antibodies or enzyme fragments thereof; b) the risk to transmit some pathogen agent, using compositions obtained from animal cells (hybridomas) (6); c) prohibitive costs of large scale antibody administration (up to some ten or hundred gram for each therapy cycle); d) the more and more limiting government rules relating to the use of the experimental animals; and e) the poor feasibility of antibody fragmenting, deriving, etc. processes when using conventional biochemical methods.

In order to ovrecome these problems, the genetic engineering and the innovative antibody technology developped techniques for identifying, amplifying and manipulating of antibody chain (H = heavy; L = light) coding sequences (7).

Different methods can be used to obtain engineered antibodies; among them, a method uses chimeric or "humanized" antibodies, wherein murine H and L variable regions are fused to human H and L constant regions; however these molecular hybrids, having a short murine sequence only (at binding sites among the antibody different domains), are able to induce an immune response of the patient and, consequently, show significant use limitations (8).

Also the global murine antibody production in plants showed a number of problems, particularly as far as quantity, correct assembly and post-translational modifications, such as the glycosilation, were concerned, that occur in amino acid sequence sites of the vegetal cell other than in animal cells (2, 9, 10).

Furthermore, though the engineered antibodies production in bacteria and/or in yeasts showed to be successful (1, 11), poor results occured when clinical applications were involved. This very probably occurs because of purifying problems of the concerned protein fraction from bacterial protein and toxin mixture, or of the selection of codons other than the preferably used codons in mammal cells, or post-translating modification processes (such as secretion and glycosilation), which operate and are regulated in bacteria or fungi otherwise than in animal cells (5, 11).

Therefore a requirement exists to make available a process to produce in plants single domain antibodies in plants (variable heavy region) or single antibody chains (variable heavy region fused to the light variable region), in order to use transgenic plants as phytoantibody biomanufacturers. These phytoantibodies are an advantageous alternative to produce useful engineered antibodies in pharmacology, as being synthesized from eukaryotic organisms, animal virus free, and being poorly immunogenic products, as having relatively simple molecules, already correctly assembled (fused VH and LH) and however still stable with a good affinity with its own antigen.

The authors (EP 92830330.4, 12) have already developped an expression vector for single domain antibodies(variable heavy region, VH-dAbs only), to be used in plants, and obtained a correct phytoantibody synthesis.

Recently the authors found surprisingly that engineered antibodies from vegetal species (Fv:variable chain or scAb: single chain antibodies) could be purified and are able to recognize the antigen both in solid and in solubilized form. The absence of the constant domains (Fc) prevents the cascade reactions of the complement, thus making the phytoantibodies mostly suitable for in vivo therapy.

Accordingly, the authors developped a process to produce single chain antibodies (variable heavy region fused with the variable light region: VH-VLscAb), showing a good binding affinity to the antigen.

The process advantageously allows to produce low cost antibodies to be used in diagnostics and therapeutics. The obtained transgenic plants produced phytoantibodies in an amount of appr. 1 % of the total protein contents of the leaf tissue, being this last the most abundant one, without showing neither physiologic nor biologic difference when compared with control plants, as far as its conventional growth and development parameters were concerned.

The authors found that these antibodies could be advantageously used in the oncology field, as being able to produce antibodies which detect the HER-2 oncogene. The HER-2 oncogene codifies for a 185 KD membrane receptor, having a tyrosine-kinase activity (13), and a transforming activity depending from its overexpression (14). In the mammary and ovary carcinomas, high HER-2 levels are unambiguously related to a fatal prognosis (15). Accordingly, an accurate measurement of said levels are essential to allow a rational disease clinical control. Moreover, some anti-gp185 antibodies show a cytostatic activity on tumor cell lines, that overexpress the receptor (16 and 17); anti-gp185 immunotoxines show a high specif cytotoxicity (LD₅₀=10⁻⁹M), indipendently from the receptor expression level or phosphorylation degree (18). This last feature could allow a perspective use of anti-gp185 immunotoxines also in neoplasias that express very poorly the receptor, e.g. the cutaneous melanoma (19).

Murine anti-gp185 antibodies, which recognize separated determinants of the extracellular receptor portion, were generated by Digiesi and al., (1992) (18) and characterized according to isotype, affinity, epitope specificity, immunohystochemical reactivity onto both fresh and fixed tissues through conventional hystopatology procedures, identification of antibodies pairs, that are suitable to be used in dual determinant type assays to measure receptor soluble forms.

It is an object of the invention a process to produce antibodies in plants for pharmacology or diagnosis purposes, comprising the steps as follows:
- to obtain an expression vector comprising the coding sequence for said antibodies;
- to transform recipients plants using said vector;
- to select antibody producing plants; and
- to purify said antibodies.

According to a preferred embodiment, said plants are of the Tabacum genus, more preferably of the Nicothiana benthamiana species.

In an embodiment, said antibodies are antibodies which are able to detect and react with the product of the HER-2 oncogene derivative, named HER-2 gp185, that is present in mammary and ovary tumors.

It is a further object of the invention the use of the said antibodies to prepare diagnostic or pharmacologic compositions.

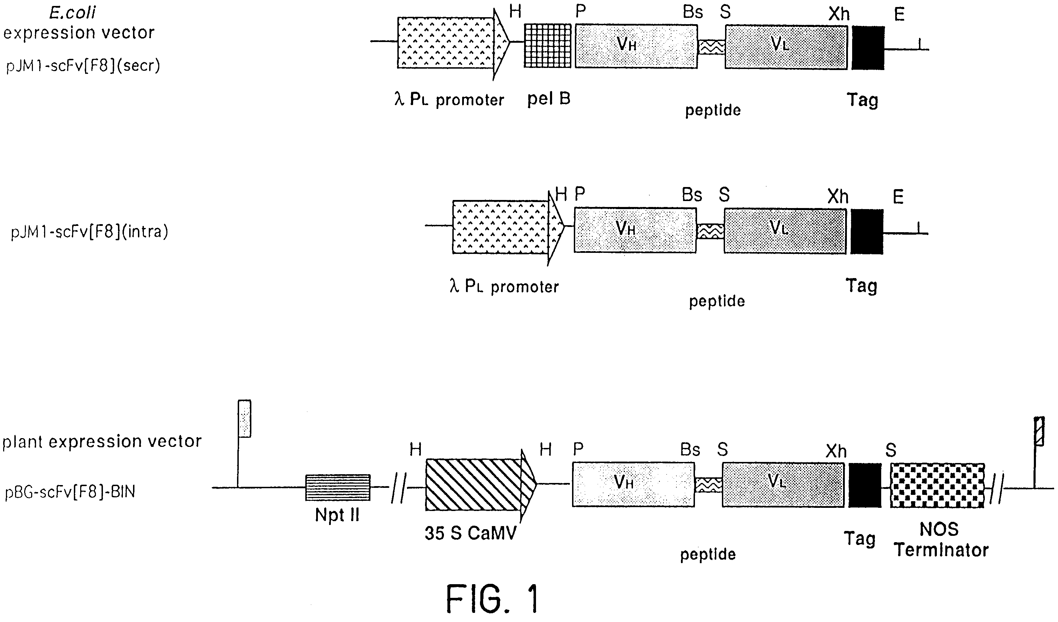

The invention will be described in the following with reference to some preferred embodiments, that do not limit in any way its scope. Particular reference will be made to the annexed fig.1, wherein a scheme of the used carriers is shown.

The gp185 anti monoclonal antibodies were obtained using conventional techniques, well known by those skilled in the art, that are described in (20) and (21) and included herein as reference. As an example, antibodies that recognize two different epitopes of gp185 are those produced by the two hybridoma lines as follows:
800E-6 IgG1 clone: affinity 8 x 10⁹ Mol/L; and
100A-4 IgG2 clone: affinity 2 x 10¹⁰Mol/L.

### RNA ISOLATION, cDNA SYNTHESIS AND VECTOR CONSTRUCTION

The RNA extraction from the hybridomas was carried out through Cell lysis in presence of guanidine thiocyanate, acid phenol extraction and isopropanol precipitation. The used protocol was in accordance with the instructions supplied by RNA Agents™ Total RNA Isolation Kit, PROMEGA.

The total RNA was appr. 1200 µg polyA⁺ RNA was isolated from a fraction of the total RNA, using the procedure as described in the manual of Sambrook and al. (22). Appr. 5 µg of purified polyA⁺ RNA were obtained.

Total messenger and polyA⁺ RNA from the hybridoma was used as template to synthesize the first cDNA strand, with reactants and reverse transcriptase, as supplied by the GeneAmp RNA PCR Kit (PERKIN ELMER CETUS). The reaction was carried out in presence of the four deoxytriphosphate nucleosides and of the reverse transcriptase, following a RNA hybridization with oligo-random, or oligo-dT or oligonucleotides specific for immunoglobulin sequences.

The more satisfactory elongation for the VH heavy chain was obtained using the CH1FOR oligonucleotides, comprising an equimolar mixture of the three oligonucleotides as follows:

MOCG12 FOR:     5'd(CTCAATTTTCTTGTCCACCTTGGTGC)3';
MOCG3 FOR:      5'd(CTCGATTCTCTTGATCAACTCAGTCT)3';
MOCM FOR:       5'd(TGGAATGGGCACATGCAGATCTCT)3'.

The following oligonucleotide was used for the light chain:

CK FOR: 5'd(CTCATTCCTGTTGAAGCTCTTGAC) 3'.

The oligonucleotides used for the amplification were as follows: 
VH1FOR:  5'd(TGAGGAGACGGTGACCGTGGTCCCTTGGCCCCG);
VH1BACK: 5'd(AGGTsmArCTGCAGsAGTwGG);
wherein: s was C or G; m was A or C; r was A or G and w was A or T;

WK1 FOR:  5'd(CCGTTTGATCTCGAGCTTGGTGCC);
VK1 BACK: 5'd(GACATCGAGCTCACTCAGTCTCCA).

The amplification primer oligonucleotides comprised the cloning useful restriction sites PstI and BstII for VH, and SacI and XhoI for VL (EP 92830330.4, 23, 24).

The amplified DNA was purified by 1.4% agarose gel, in accordance to the standard procedures as suggested by the Geneklean Kit (Bio 101) protocol.

The amplified and purified DNA was used for a ligase reaction (ligase 10 U/µl, PROMEGA) with the intermediate plasmids (modified pUC19, Ward and al. (1) and pGEM 11Zf+, PROMEGA), digested respectively with PstI and BstEII for VH and SacI and XhoI for VL. After having been allowed to react for 3-4 hours at room temperature, plasmids were transformed in E.coli, DH5α strain, which was made competent according to the process herebelow: bacteria were grown under stirring overnight at 37°C, diluted in a fresh culture medium and then grown under stirring at 37°C up to the 0.2 absorbance at 600 nm. Then bacteria were centrifuged at 4,000 rpm for 15 minutes at 4°C; resuspended in one tenth of the starting volume with CaCl₂, 50 mM; ice frozen at least for 30 minutes; resuspended in one hundredth of the starting volume with CaCl₂, 50 mM; and finally ice frozen up to the transformation, that was carried out in accordance with the protocol of Sambrook and al. (22).

Then bacteria colonies were analyzed with restriction enzymes on DNA minipreparations, according to standard procedures. Positive clones were grown in order to obtain a plasmid large amount to be used for further restriction analyses and cloning steps.

The pJM1scFv plasmid (Dr. G. Winter's gift), comprising the (Gly₄Ser)₃ linker sequence, included the VH and VL regions of the anti-HER-2 antibody. The pJM1scFv plasmid was grown in EMG5578 Escherichia coli (24) and digested with HindIII/EcoRI; the obtained fragment was inserted in the plant expression vector pBG-dAb using the same sites. This vector is derived from the pBG-dAb-BIN vector, described in EP 92030330.4, and deposited at NCAIM under the No. 001144. A scheme is shown in fig.1. Accordingly, the pBG-dAb anti-HER-2 vector was obtained.

The product of the ligase reaction as previously described was analyzed for restriction and the useful plasmid to transform plants was grown in a large amount according to standard protocols. The pBG-BIN-dAb plant expression vector comprised: a NptII (neomycin phosphotranferase II) marker to select the transgenic plants; the (left and right) T-DNA sequences, that are able to transfer the DNA from the Agrobacterium vector to plant cells; a constitutive promoter (CAMV35S) of Cauliflower Mosaic Virus; the useful restriction sites to insert any antibody variable region; a phytoantibody fused TAG sequence, that codified only 11 c-myc derived amino acids, which could be detected by the SE10 monoclonal antibody; a termination of the transcription NOS sequence (deriving from the gene for plant nopaline synthetase); and a signal for plant mRNA polyadenilation.

### PROTOPLAST PREPARATION AND TRANSFORMATION TO TRANSIENTLY EXPRESS THE ANTIBODY

Protoplasts from N. benthamiana plants were transformed to express transiently the anti-HER2 gp185 engineered antibody, in order to assay preliminarly the binding affinity to the antigen, as well as to develop labelling procedures using radio isotopes, toxines, etc. Protoplasts were obtained using a protocol as follows: leaves from young plants (2-3 weeks in vitro) were picked up and cut in small fragments under sterility conditions. Fragments were slowly stirred overnight in the darkness at 20°C into a solution of Driselase (plant tissue culture medium, SIGMA), 0.1% at a 1g:10ml ratio. Free protoplasts from cell wall, after having been filtered through sterile steel filters (200 mesh, SIGMA), were collected and then centrifuged for 10 minutes RT at 1000 rpm. The pellet was resuspended in a solution comprising sucrose, 0.6 M, allowing the protoplasts to float after having been centrifuged at 1000 rpm for 10 min. RT. Recovered protoplasts were washed with a solution comprising 0.1% mannitole, 0.5 M MES, pH 5.6. Purified protoplasts as above were accounted and diluted to a range from 10⁶ up to 10⁷ cells/ml.

The protoplast (10⁶-10⁷ cells/ml together with 10 µg of the DNA of the pBG-dAb anti-HER2 expression vector) transformation was carried out in presence of 0.5 ml of PEG (PEG 6000, MERCK), 30%, for 0.5-1 min. at room temperature, followed by a dilution with 5 ml of mannitol, 0.5 M MES, 0.1%, 3 mM of CaCl₂, pH 5.6. Then transformed protoplasts were made free from the PEG excess using the same solution as above, and collected in Petri's dishes for some hours in a climatic chamber before the transfer to the nutrient medium. The protoplast growth (48 hours in a climatic chamber) was carried out in a medium, comprising MS (Murashige and Skoog Basal Medium, SIGMA) salts, CaCl₂, xylose, sucrose, mannitol, vitamins and hormones, as described for tobacco protoplasts (25). When the growth was completed, total proteins were extracted using an isotonic buffer (added with anti-protease, as described in 24) and the obtained engineered antibody purified.

### ENGINEERED ANTIBODY (ANTI-HER2gp185) AFFINITY ASSAY

After having been grown the transformed protoplasts with the expression vector, the engineered antibody was extracted and purified through an affinity chromatography procedure. A TAG sequence (derived from the c-myc oncogene), which is recognized from the so called 9E10 (1 and 26) monoclonal antibody, was present at the carboxyl terminal of the obtained construct. The phytoantibody was assayed through ELISA and immunohistochemical assays (27) for antigen detection. Furthermore, a number of conveniently modified (ammonium sulfate, caprilic acid, etc.) physico-chemical treatments were carried out, as well as ion exchange (DEAE) and affinity chromatography assays on 9E10 anti-TAG, that also could be used to large scale purification process.

### TRANSFORMATION OF NICOTIANA PLANT LEAF DISKS WITH AGROBACTERIUM

The Agrobacterium plant transformation and regeneration was carried out in accordance with the protocol of Tavazza, R. and al (28). The leaf disk transformation was obtained through a co-cultivation with Agrobacterium. The transgenic plant regeneration was carried out in a selective medium.

### MOLECULAR ANALYSIS OF TRANSGENIC PLANT

Plants, which are able to regenerate in a selective medium (i.e., to generate roots and leaves and to appear as a whole plant after having been transformed), were analyzed through a Northern procedure (i.e. total RNA from leaf analysis using a vector derived, molecular probe); then positive plants were analyzed by Western blot after having the antibody been extracted and purified from total leaf proteins.

### COMPARATIVE ANALYSIS BETWEEN THE ANTI-HER2gp185 AND MONOCLONAL ANTIBODY ACTIVITIES

Both reactants (i.e., engineered and monoclonal antibody) could be analyzed in parallel for their reactivities against normal and neoplastic tissues, as well as their behavior when subjected to immunochemical assays (such as immunoprecipitation, RIA or ELISA analyzed by scatchard-plot (20, 22, 27, 29 and 30)).

### SOLUBLE ANTIGEN ASSAY DEVELOPMENT

The engineered antibody could be bound to a solid matrix in order to develop double determinant assays, as described in the case of natural antibodies (18, 20, 21 and 27).

### ASSAYS TO DEFINE THE BIOLOGIC ACTIVITY OF THE ENGINEERED ANTIBODY

An ELISA assay could be carried out on whole cells (27), using the anti-TAG9E10 monoclonal antibody as secondary antibody and a polyclonal antibody anti-murine IgG as tertiary antibody. This assay was used as general probing assay and as to following process of the antibody activity during the purifying procedures. An indirect immunofluorescence assay could be carried out using a tertiary fluoresceine or rodamine antibody. An indirect immunoprecipitation (27) could be carried out, using the engineered antibody covalently bound to an inert matrix, such as agarose or sepharose.

The engineered antibody resistance against a radio labelling procedure with ¹²⁵I (chloramine T, Iodogen, BOLTON HUNTER) could be tested in presence of an antigen excess (20 and 27).

The maintained antibody specifity after each purifying or manipulating process (e.g. iodination, see above) was tested through checking that the binding ability was in accordance with the typical conventional criteria, such as competition (between antigene and the specific antibody), saturation, titration and reversion (20, 21 and 27).

All these assays used cell lines expressing the HER-2gp185 oncogene to a more or a less extent, such as for instance NIH-3T3 infected lines through a translation process according to standard method using HER-2.

### REFERENCES

(1) Ward, E.S. and al.; "Nature", 341, 544-546 (1989).
(2) Hiatt, A., Cafferkey, R. and Bowdish, K.; "Nature", 342, 76-78 (1989).
(3) Hiatt, A.; "Nature", 344, 469-470 (1990).
(4) Borrebaeck C.A.K. Editor: "Antibody Engineering - A Practical Guide", Reeman W.H. and Co., New York (1992).
(5) Nisticb, P. and al.; "J. Cell. Invest.", 90, 1093-1099 (1992).
(6) Wilkinson R.; "Art to Science", 12, 1-5 (1993).
(7) Winter, G. and Milstein, C.; "Nature", 349, 293-299 (1991).
(8) Orlandi, R.; "Biotec.", 5, 46-49 (1990).
(9) During, K. and al.; "Plant Mol.Biol.", 15, 281-293, (1990).
(10) Hiatt, A., Whelan W.J. Eds., "Miami Biotechnology Winter Symposium (1992).
(11) Horwitz, A.H. and al.; "Proc.Natl.Aacad.Sci.USA", 85, 8678-8682 (1988).
(12) Benvenuto, E. and al. "Plamt. Molecular Biology", 17, 865-874 (1991).
(13) Schechter, A.L. and al. "Nature", 312, 513-516 (1984).
(14) Di Fiore, P.P. and al.; "Science", 237, 178-182 (1987).
(15) Slamon, D.J. and al.; "Science", 224, 707-712 (1989).
(16) Hudziak, L.A. and al.; "Mol. and Cell. Biology", 9, 1165-1172 (1989).
(17) Hudziak, L.A. and al.; "Cancer Res.", 51, 5361-5369 (1991).
(18) Digiesi, G. and al. "Hybridoma", 11, 519-527 (1992).
(19) Natali, P.G. and al. Paper in preparation (1993).
(20) Giacomini, P., Natali, P.G. and Ferrone S.; "J. of Immunol.", 135, 696 (1985).
(21) Giacomini, P. and al; "Cancer Res.", 43, 3586 (1983).
(22) Sambrook J, Fritsch, E.F., Maniatis, T.; "Molecular Cloning", Cold Spring Harbor Laboratory, New York (1989).
(23) Orlandi, R. and al. "Proc. Natl. Acad. Sci. USA", 86, 3833-3837 (1989).
(24) Tavladoraki et al.; "Nature" under printing (1993).
(25) "Handbook of Plant Cell Culture", Eds. Evans, Sharp Ammirato, McMillan Pub. (1986).
(26) Muro S. and Pelham H.; "Cell", 46, 291-300 (1986).
(27) Giacomini, P., Segatto O and Natali, P.G.; "Int.J. Cancer", 39, 729-736 (1987).
(28) Tavazza, R. and al.; "J. Plant Pysiol.", 133, 640-644 (1988).
(29) Gelvin, S.B., Schilperoort, R.A. and Verma D.P.S. (eds.); "Plant Molecular Biology", Manual Kluwer Academic Publishers (Dordrecht, Boston, London), 2nd edition (1989).
(30) Draper, J, Scott, R., Armitage, P. and Walden R. (eds.), "Plant Genetic Transformation and Gene Expression, A Laboratory Manual", Blackwell Scientific Publications, Oxford (1988).

## Claims

1. Process to produce antibodies in plants to be used in pharmacology or diagnostics, comprising the steps as follows:
- to construct an expression vector comprising the codifying sequence of said antibodies;
- to transform recipient plants using said vector;
- to select plants that are able to produce said antibodies; and
- to purify said antibodies.

2. Process to produce antibodies in plants to be used in pharmacology or diagnostics according to claim 1, wherein said plants belong to the Tabacum genus.

3. Process to produce antibodies in plants to be used in pharmacology or diagnostics according to claim 2, wherein said plants belong to the Nicothiana benthamiana species.

4. Process to produce antibodies in plants to be used in pharmacology or diagnostics according to any of previous claims, wherein said antibodies is are able to recognize, and to react with the HER-2 oncogene product, as HER-2gp185, that is present in mammary and ovary tumors.

5. Use of the antibodies produced according to any of previous claims to obtain diagnostics or pharmacology compositions.
